# EUROPÄISCHE PATENTSCHRIFT

(11) **EP 1 960 017 B1**
(45) Veröffentlichungstag und Bekanntmachung des Hinweises auf die Patenterteilung: **02.11.2011**
(21) Anmeldenummer: 06754727.3
(22) Anmeldetag: 14.07.2006
(51) Int. Cl.: A61M 1/36

(54) **VERFAHREN ZUM FREIBLASEN EINES BENETZTEN HYDROPHOBFILTERS UND VORRICHTUNG ZUR DURCHFÜHRUNG DES VERFAHRENS**
METHOD FOR BLOWING FREE A WETTED HYDROPHOBIC FILTER, AND DEVICE FOR CARRYING OUT THE METHOD
PROCÉDÉ DE PURGE D'UN FILTRE HYDROPHOBE MOUILLÉ ET DISPOSITIF DE RÉALISATION DU PROCÉDÉ

(30) Priorität: 05.12.2005 DE 102005058012
(43) Veröffentlichungstag der Anmeldung: 27.08.2008
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE)
(74) Vertreter: Laufhütte, Dieter
(86) Internationale Anmeldenummer: PCT/EP2006/006912
(87) Internationale Veröffentlichungsnummer: WO 2007/065490

(56) Entgegenhaltungen:
- EP-A- 0 330 761
- EP-A- 1 319 417
- EP-A- 1 547 630
- DE-A1- 3 543 126
- US-A- 3 964 479
- US-A- 5 643 455

## Beschreibung

Die Erfindung betrifft ein Verfahren zum Freiblasen eines benetzten Hydrophobfilters und eine Vorrichtung zur Durchführung dieses Verfahrens.

Es ist bereits bekannt, in einem extrakorporalen Blutkreislauf einen Drucksensor über eine Schlauchleitung, die von diesem Blutkreislauf abzweigt, anzuschließen, um den Druck im Blutkreislauf zu messen. Grundsätzlich kann diese Anschlussleitung an einer beliebigen Stelle des Blutkreislaufes, beispielsweise von einer im Blutkreislauf vorhandenen venösen Luftabscheidekammer abzweigen.

In dem abzweigenden Schlauch, an welchem der Drucksensor angeschlossen ist, ist eine bestimmte Menge Luft eingeschlossen, über die der Druck zum Drucksensor übertragen wird. Üblicherweise ist der Drucksensor in einer Blutbehandlungsmaschine integriert. Um das unerwünschte Vordringen von Blut bis in die Blutbehandlungsmaschine zu verhindern, wird mindestens ein hydrophober Schutzfilter in der Leitung angeordnet. Dieser Hydrophobfilter wird zusammen mit dem restlichen Blutschlauchset des extrakorporalen Blutkreislaufs nach einer erfolgten Blutbehandlung verworfen.

Während des Betriebs des extrakorporalen Blutkreislaufs kann es vorkommen, dass der Hydrophobfilter vom Blut benetzt wird und dadurch einseitig zumindest teilweise verstopft. Hieraus folgt - teilweise schleichend - eine Beeinträchtigung der Funktionsfähigkeit der Druckmessung. Mit diesem Problem setzt sich die EP-A-0 330 761 auseinander. Um den Fehlzustand erkennen zu können, soll nach dieser Lehre von dem Drucksensor nicht nur der statische Druck erfaßt werden, es sollen auch die in dem extrakorporalen Blutkreislauf vorhandenen Druckschwankungen erfaßt werden. Soweit das Drucksensorsystem unempfindlicher wird, wird nicht nur der statische Druck nicht mehr korrekt angezeigt, sondern auch die periodischen Druckschwankungen werden nicht mehr wiedergegeben. Falls also die Druckschwankungen hier durch den Drucksensor nicht mehr regelmäßig festgestellt werden können, kann darauf zurückgeschlossen werden, dass der Hydrophobfilter so weitgehend verstopft ist, dass keine hinreichend genaue Druckmessung mehr möglich ist.

Soweit der Anwender dieses Problem erkennt, kann er das Filter kontrolliert zerstören und durch einen zweiten Filter ergänzen. In der Regel ist aber der Hydrophobfilter mit dem gesamten Blutschlauchset verbunden, so dass ein Filterwechsel nur unter erheblichem Aufwand möglich ist.

Aus der US-A-3,964,479 ist bereits ein extrakorporaler Blutkreislauf bekannt, bei dem ein Drucksensor an einem Abzweig einer im Blutkreislauf vorgesehenen Luftabscheidekammer anschließt. Die Lehre dieses US-Patentes beschäftigt sich mit der Problematik, Flüssigkeitspegel innerhalb der Luftabscheidekammer in einer gewünschten Höhe einzustellen. Hierzu wird von der Leitung zwischen der Luftabscheidekammer und dem Drucksensor, in der auch nach der bekannten Lösung ein Hydrophobfilter vorgesehen ist, eine Luftpumpe abgezweigt, die dazu dient, den Flüssigkeitspegel in der Luftabscheidekammer auf ein bestimmtes Niveau zu setzen. Hierzu kann Luft in die Luftabscheidekammer gepumpt bzw. aus jener abgezogen werden.

Aufgabe der vorliegenden Erfindung ist es, ein Verfahren bzw. eine Vorrichtung vorzuschlagen, die bei Feststellung eines Verstopfens des Filters, diese Fehlfunktion möglichst automatisch beseitigt und zumindest dazu führt, dass die Hydrophobfilter weniger häufig gewechselt werden müssen.

Erfindungsgemäß wird diese Aufgabe durch die Kombination der Merkmale des Anspruchs 1 gelöst. Erfindungsgemäß wird hier ein Verfahren zum Freiblasen eines benetzten Hydrophobfilters in einem extrakorporalen Blutkreislauf geschaffen, wobei der extrakorporale Blutkreislauf eine Luftabscheidekammer aufweist, an die über eine Leitung ein Druckaufnehmer und eine Luftpumpe angeschlossen sind, wobei der Hydrophobfilter in dieser Leitung angeordnet ist. Das erfindungsgemäße Verfahren umfasst zumindest folgende Schritte:
- Überwachen der Luftdurchlässigkeit des Hydrophobfilters und
- Freiblasen des Hydrophobfilters mittels der angeschlossenen Luftpumpe, falls festgestellt wird, dass der Hydrophobfilter verstopft ist.

Die Überwachung der Luftdurchlässigkeit des Hydrophobfilters kann beispielsweise analog bereits der zuvor mitgeteilten Lehre gemäß der EP-A-0 330 761 erfolgen. Wird eine zumindest teilweise Verstopfung des Hydrophobfilters festgestellt, wird über eine entsprechende Steuerung die angeschlossene Luftpumpe derart angesteuert, dass der Hydrophobfilter freigeblasen wird.

Diese erfindungsgemäße Lehre erfährt in den Unteransprüchen eine bevorzugte Ausgestaltung.

Demnach kann die Luftdurchlässigkeit des Hydrophobfilters mittels des Druckaufnehmers überwacht werden.

Während des Freiblasens des Hydrophobfilters kann einerseits eine Klemme, die im extrakorporalen Blutkreislauf stromab der Luftabscheidekammer angeordnet ist, und können andererseits Ventile in den Verbindungsleitungen zur Luftpumpe und zum Druckaufnehmer geöffnet werden.

Vorteilhaft wird der Druckverlauf über die Zeit während des Freiblasens durch die Luftpumpe aufgenommen.

Überschreitet hier die Druckveränderung pro Zeiteinheit, also die Druckveränderungsrate, einen vorgegebenen Grenzwert, wird ein Alarmsignal zum Auswechseln des Hydrophobfilters gegeben. Hier steht dann fest, dass ein Freiblasen ohne weiteres nicht möglich ist. Dabei ist zu berücksichtigen, dass die Blasgeschwindigkeit der Luftpumpe nicht beliebig erhöht werden kann, da im Grenzfall der Berstdruck des Hydrophobfilters überschritten wird.

Falls allerdings die Druckveränderung pro Zeiteinheit den vorgegebenen Grenzwert nicht überschreitet, kann nach Freiblasen des Filters das Ventil in der Zuleitung der Luftpumpe vorteilhaft wieder geschlossen werden.

Gemäß einer anderen vorteilhaften Ausgestaltung der Erfindung wird während des Freiblasens die Füllhöhe des Bluts in der Luftabscheidekammer mittels eines Füllhöhendetektors überwacht. Hierdurch kann verhindert werden, dass die Füllhöhe zu weit absinkt und im Extremfall Luft in das Schlauchsystem und damit in den Patienten gelangt.

Vorteilhaft ist es, wenn die Luftpumpe mittels eines Steuer- und Überwachungsprogramms angesteuert wird, das gleichzeitig die Druckwerte über die Zeit aufzeichnet.

Eine Vorrichtung zur Durchführung des erfindungsgemäßen Verfahrens ist in Anspruch 9 wiedergegeben. Diese Vorrichtung weist eine in einem extrakorporalen Blutkreislauf angeordnete Luftabscheidekammer auf, an die über eine Leitung ein Druckaufnehmer und eine Luftpumpe zum Einstellen des Füllstandes in der Luftabscheidekammer anschließen, wobei in dieser Leitung ein Hydrophobfilter angeordnet ist. Zur Durchführung des vorgenannten Verfahrens weist diese Vorrichtung eine Steuer- und Überwachungseinheit auf, über die die Luftpumpe zum Freiblasen des Hydrophobfilters ansteuerbar ist, während gleichzeitig die Druckwerte pro Zeiteinheit aufnehmbar und überwachbar sind.

Weiterhin umfasst die Vorrichtung zur Durchführung des erfindungsgemäßen Verfahrens vorzugsweise eine Alarmvorrichtung zur Ausgabe eines Alarms für den Fall, dass der Hydrophobfilter nicht freiblasbar ist.

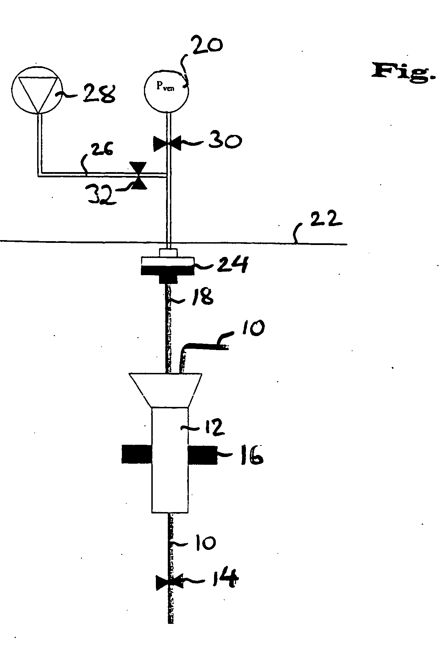

Weitere Merkmale, Einzelheiten und Vorteile der Erfindung werden anhand des in der einzigen Figur beigefügten Ausführungsbeispiels erläutert. Die einzige Figur zeigt eine schematische Darstellung eines Teils eines extrakorporalen Blutkreislaufs.

Von einem extrakorporalen Blutkreislauf ist in der Darstellung gemäß der Figur nur ein Teil eines blutführenden Schlauchs 10 gezeigt, der in eine konventionell aufgebaute Luftabscheidekammer 12 von oben hineinführt und am unteren Ende der Luftabscheidekammer wieder anschließt. Die Luftabscheidekammer kann z. B. in der Blutrückgabeleitung (venöse Blutleitung) des extrakorporalen Blutkreislaufs angeordnet sein. Unterhalb der Luftabscheidekammer 12 ist im blutführenden Schlauch 10 eine Schlauchklemme 14 angeordnet. Zur Erfassung der Füllhöhe des Bluts innerhalb der Luftabscheidekammer 12 ist ein Füllhöhendetektor 16 an der Luftabscheidekammer angeordnet.

Die Luftabscheidekammer 12 ist über einen luftführenden Schlauch 18 mit einem Drucksensor 20 verbunden. Der Drucksensor 20 ist innerhalb einer Dialysevorrichtung angeordnet, deren Außenwand hier aus Vereinfachungsgründen nur durch den Strich 22 angedeutet ist. In der Leitung 18 zwischen der Luftabscheidekammer 12 und dem Drucksensor 20 ist noch außerhalb des Dialysegeräts ein Hydrophobfilter 24 angeordnet.

Von der luftführenden Leitung 18 zweigt innerhalb des Dialysegeräts eine Leitung 26 ab, die zu einer Luftpumpe 28 führt. Die Leitung 18 weist zwischen dem Abzweig zur Leitung 26 und dem Drucksensor 20 ein steuerbares Ventil 30 auf. Die luftführende Leitung 26 weist ebenfalls ein steuerbares Ventil 32 auf.

Während des üblichen Betriebs des extrakorporalen Blutkreislaufs tropft Blut aus der Leitung 10 in die Luftabscheidekammer 12. Der Füllstand in der Luftabscheidekammer 12 wird mittels des Füllhöhendetektors 16 überwacht. Sollte er unter ein gewünschtes Niveau fallen, kann hier über die Luftpumpe 28 oder die Klemme 14 die Höhe des Füllstandes des Bluts in der Luftabscheidekammer 12 eingestellt werden. Während des Betriebs des extrakorporalen Blutkreislaufs wird der Druck durch den Drucksensor 20 bestimmt. Über ein Messroutine, wie sie beispielsweise aus der EP-A-0 330 761 bekannt ist, kann hier festgestellt werden, ob der Hydrophobfilter 24 durch Benetzung mit Blut zumindest teilweise verstopft ist. Wird eine teilweise Verstopfung festgestellt, werden die Ventile 30 und 32 geöffnet (falls das Ventil 30 nicht schon geöffnet war) und es wird mittels der Luftpumpe 28 ein Überdruck erzeugt, um den Hydrophobfilter 24 freizublasen. Während dieses Freiblasens wird gleichzeitig die Schlauchklemme 14 geöffnet (falls sie nicht schon geöffnet war), um einen Überdruck im System zu vermeiden.

Während der Beaufschlagung des Hydrophobfilters 24 mit Luft, wird der Druckverlauf mittels des Drucksensors über die Zeit überwacht. Sollte der Druck zu schnell ansteigen, deutet dies auf ein grundsätzliches Problem im Filter hin, das durch ein Freiblasen mittels der Luftpumpe 28 nicht behoben werden kann. In diesem Fall wird von der Steuer- und Überwachungseinrichtung ein Alarmsignal aktiviert, das über eine entsprechende Alarmvorrichtung an den Anwender ausgegeben wird. Beim Alarmfall muss der Hydrophobfilter 24 ausgewechselt werden.

Sollte jedoch der Druckanstieg ausreichend gering sein, ist ein Freiblasen möglich. In diesem Fall muss lediglich noch durch den Höhendetektor 16 sichergestellt werden, dass der Blutspiegel innerhalb der venösen Luftabscheidekammer 12 nicht zu weit absinkt um ein Eindringen von Luft in das Schlauchsystem 10 innerhalb der Luftabscheidekammer 12 sicher zu verhindern.

## Patentansprüche

1. Verfahren zum Freiblasen eines benetzten Hydrophobfilters in einem extrakorporalen Blutkreislauf mit einer Luftabscheidekammer, an die über eine Leitung ein Druckaufnehmer und eine Luftpumpe angeschlossen sind, wobei der Hydrophobfilter in dieser Leitung angeordnet ist, mit folgenden Schritten:
- Überwachen der Luftdurchlässigkeit des Hydrophobfilters und
- Freiblasen des Hydrophobfilters mittels der angeschlossenen Luftpumpe, falls festgestellt wird, dass der Hydrophobfilter verstopft ist.

2. Verfahren nach Anspruch 1, **dadurch gekennzeichnet, dass** die Luftdurchlässigkeit des Hydrophobfilters mittels des Druckaufnehmers überwacht wird.

3. Verfahren nach Anspruch 1 oder Anspruch 2, **dadurch gekennzeichnet, dass** während des Freiblasens des Hydrophobfilters einerseits eine Klemme, die im extrakorporalen Blutkreislauf stromab der Luftabscheidekammer angeordnet ist, und andererseits Ventile in den Verbindungsleitungen zur Luftpumpe und zum venösen Druckaufnehmer geöffnet werden.

4. Verfahren nach Anspruch 3, **dadurch gekennzeichnet, dass** der Druckverlauf über die Zeit während des Freiblasens durch die Luftpumpe aufgenommen wird.

5. Verfahren nach Anspruch 4, **dadurch gekennzeichnet, dass** für den Fall, dass die Druckveränderung pro Zeiteinheit einen vorgegebenen Grenzwert überschreitet, ein Alarmsignal zum Auswechseln des Hydrophobfilters gegeben wird.

6. Verfahren nach Anspruch 4, **dadurch gekennzeichnet, dass** für den Fall, dass die Druckveränderung pro Zeiteinheit einen vorgegebenen Grenzwert nicht überschreitet, nach Freiblasen des Filters das Ventil in der Zuleitung der Luftpumpe wieder geschlossen wird.

7. Verfahren nach einem der Ansprüche 1 bis 6; **dadurch gekennzeichnet, dass** während des Freiblasens die Füllhöhe des Bluts in der Luftabscheidekammer mittels eines Füllhöhendetektors überwacht wird.

8. Verfahren nach einem der Ansprüche 1 bis 7, **dadurch gekennzeichnet, dass** die Luftpumpe mittels eines Steuer- und Überwachungsprogramms angesteuert wird, das gleichzeitig die Druckwerte über die Zeit aufzeichnet.

9. Vorrichtung zur Durchführung eines Verfahrens nach einem der Ansprüche 1 bis 8 mit einer in einem extrakorporalen Blutkreislauf angeordneten Luftabscheidekammer (12), an die über eine Leitung ein Druckaufnehmer (20) und eine Luftpumpe (28) anschließen, wobei in dieser Leitung ein Hydrophobfilter (24) angeordnet ist,
**gekennzeichnet durch,**
eine Steuer- und Überwachungseinheit, über die die Luftpumpe (28) zum Freiblasen des Hydrophobfilters (24) ansteuerbar ist, während gleichzeitig die Druckwerte pro Zeiteinheit aufnehmbar und überwachbar sind.

10. Vorrichtung nach Anspruch 9, **gekennzeichnet durch** eine Alarmvorrichtung zur Ausgabe eines Alarms für den Fall, dass der Hydrophobfilter nicht freiblasbar ist.

## Claims

1. A method of blowing free a wetted hydrophobic filter in an extracorporeal blood circuit with an air separation chamber to which a pressure pick-up and an air pump are connected via a conduit, the hydrophobic filter being disposed in said conduit, comprising the following steps:
- monitoring the air permeability of the hydrophobic filter, and
- blowing free the hydrophobic filter by means of the connected air pump if it is detected that the hydrophobic filter is clogged.

2. A method in accordance with claim 1, **characterized in that** the air permeability of the hydrophobic filter is monitored by means of the pressure pick-up.

3. A method in accordance with claim 1 or claim 2, **characterized in that** while blowing free the hydrophobic filter, a clamp disposed in the extracorporeal blood circuit downstream of the air separation chamber is arranged on the one hand, and on the other hand, valves in the connecting conduits to the air pump and to the venous pressure pick-up are opened.

4. A method in accordance with claim 3, **characterized in that** the course of pressure over time is picked up during the blowing free by the air pump.

5. A method in accordance with claim 4, **characterized in that** for the case that the change in pressure per unit time exceeds a predefined limit value, an alarm signal is produced for replacing the hydrophobic filter.

6. A method in accordance with claim 4, **characterized in that** for the case that the change in pressure per unit time does not exceed a predefined limit value, the valve in the supply conduit of the air pump is closed again during the blowing free of the filter.

7. A method in accordance with one of the claims 1 to 6, **characterized in that** during the blowing free, the filling level of the blood in the air separation chamber is monitored by means of a filling level detector.

8. A method in accordance with one of the claims 1 to 7, **characterized in that** the air pump is actuated by means of a control and monitoring program which simultaneously records the pressure values over time.

9. An apparatus for performing a method in accordance with one of the claims 1 to 8, comprising an air separation chamber (12) disposed in an extracorporeal blood circuit, to which a pressure pick-up (20) and an air pump (28) are connected via a conduit, a hydrophobic filter (24) being disposed in said conduit,
**characterized by**
a control and monitoring unit via which the air pump (28) can be actuated for blowing free the hydrophobic filter (24) while at the same time the pressure values per unit time can be picked up and monitored.

10. An apparatus in accordance with claim 9, **characterized by** an alarm device for producing an alarm for the case that the hydrophobic filter cannot be blown free.

## Revendications

1. Procédé destiné à la purge d'un filtre hydrophobe mouillé dans un circuit sanguin extracorporel avec une chambre de dégagement d'air, à laquelle sont raccordés par l'intermédiaire d'une conduite un capteur de pression et une pompe à air, le filtre hydrophobe étant disposé dans cette conduite, avec les étapes suivantes :
- surveillance de la perméabilité à l'air du filtre hydrophobe et
- purge du filtre hydrophobe à l'aide de la pompe à air raccordée, s'il est constaté que le filtre hydrophobe est bouché.

2. Procédé selon la revendication 1, **caractérisé en ce que** la perméabilité à l'air du filtre hydrophobe est surveillée à l'aide du capteur de pression.

3. Procédé selon la revendication 1 ou la revendication 2, **caractérisé en ce que** pendant la purge du filtre hydrophobe, d'une part, une pince qui est disposée dans le circuit sanguin extracorporel en aval de la chambre de dégagement d'air, et d'autre part des soupapes, sont ouvertes dans les conduites de liaison vers la pompe à air et vers le capteur de pression veineuse.

4. Procédé selon la revendication 3, **caractérisé en ce que** l'allure de la pression est captée sur la période pendant la purge par la pompe à air.

5. Procédé selon la revendication 4, **caractérisé en ce que** pour le cas où la modification de pression par unité de temps dépasse une valeur limite prescrite, un signal d'alarme est émis pour le remplacement du filtre hydrophobe.

6. Procédé selon la revendication 4, **caractérisé en ce que** pour le cas où la modification de pression par unité de temps ne dépasse pas une valeur limite prescrite, la soupape est refermée dans la conduite d'amenée de la pompe à air après la purge du filtre.

7. Procédé selon une quelconque des revendications 1 à 6, **caractérisé en ce que** pendant la purge, la hauteur de remplissage du sang dans la chambre de dégagement d'air est surveillée à l'aide d'un détecteur de hauteur de remplissage.

8. Procédé selon une quelconque des revendications 1 à 7, **caractérisé en ce que** la pompe à air est démarrée à l'aide d'un programme de commande et de surveillance, qui enregistre en même temps les valeurs de pression sur la période.

9. Dispositif destiné à l'exécution d'un procédé selon une quelconque des revendications 1 à 8 avec une chambre de dégagement d'air (12) disposée dans un circuit sanguin extracorporel, à laquelle sont raccordés par une conduite un capteur de pression (20) et une pompe à air (28), un filtre hydrophobe (24) étant disposé dans cette conduite,
**caractérisé par**
une unité de commande et de surveillance, par laquelle la pompe à air (28) est démarrable pour la purge du filtre hydrophobe (24), pendant que les valeurs de pression sont captables et surveillables simultanément par unité de temps.

10. Dispositif selon la revendication 9, **caractérisé par** un dispositif d'alarme destiné à l'émission d'une alarme pour le cas où le filtre hydrophobe n'est pas purgeable.
